(19) Europäisches Patentamt
European Patent Office
Office européen des brevets

(11) Veröffentlichungsnummer: **0 447 950 B1**

(12) **EUROPÄISCHE PATENTSCHRIFT**

(45) Veröffentlichungstag der Patentschrift: **24.08.94**

(51) Int. Cl.5: **C07D 261/18**

(21) Anmeldenummer: **91103889.1**

(22) Anmeldetag: **14.03.91**

# (54) Verfahren zur Herstellung von Isoxazol-4,5-dicarbonsäure-diestern.

(30) Priorität: **21.03.90 DE 4009028**

(43) Veröffentlichungstag der Anmeldung:
**25.09.91 Patentblatt 91/39**

(45) Bekanntmachung des Hinweises auf die Patenterteilung:
**24.08.94 Patentblatt 94/34**

(84) Benannte Vertragsstaaten:
**BE CH DE DK ES FR GB IT LI NL**

(56) Entgegenhaltungen:

**EP-A- 0 421 226**

**TETRAHEDRON, Band 30, 1974, Seiten 1365-1371, Pergamon Press, Oxford, GB; A.McKILLOP et al.: "An investigation of the reaction of primary nitroalkanes withacetic anhydride/sodium acetate"**

**SYNTHESIS, Nr. 6, 1982, Seiten 508-509, Georg Thieme Verlag, Stuttgart, DE; G.A. LEE: "A simplified synthesis of unsaturated synthesis of unsaturatednitrogen-heterocycles using nitrile betaines"**

(73) Patentinhaber: **BASF Aktiengesellschaft**
**Carl-Bosch-Strasse 38**
**D-67063 Ludwigshafen (DE)**

(72) Erfinder: **Kuekenhoehner, Thomas, Dr.**
**Seidelstrasse 2**
**W-6710 Frankenthal (DE)**
Erfinder: **Maywald, Volker, Dr.**
**Berner Weg 24**
**W-6700 Ludwigshafen (DE)**

**EP 0 447 950 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Isoxazol-4,5-dicarbonsäure-diestern der allgemeinen Formel I

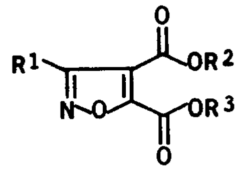

(I)

in der $R^1$ ein beliebiger, unter den Reaktionsbedingungen inerter organischer Rest ist und $R^2$ und $R^3$ jeweils für eine Alkyl-, Cycloalkyl- oder Benzylgruppe stehen.

Weiterhin betrifft die Erfindung neue Isoxazolderivate, die als Zwischenprodukte in organischen Synthesen, insbesondere zur Herstellung von Pflanzenschutzmitteln dienen.

Es ist literaturbekannt, daß Isoxazol-4,5-dicarbonsäurediester durch 1,3-dipolare Cycloaddition von Nitriloxiden IV an aktivierte Dreifachbindungen, z.B. Acetylendicarbonsäurediestern, gemäß der folgenden Reaktionsgleichung (a) hergestellt werden können (siehe z.B. Chem. Pharm. Bull. 26, 3254-3256, 1978). Die entsprechende Umsetzung mit aktivierten Doppelbindungen liefert dagegen die Isoxazolin-4,5-dicarbonsäure-diester gemäß Reaktionsgleichung (b) (siehe z.B. Bull. Chem. Soc. Jpn. 59, 2827-2831 (1986) sowie 57, 2531-2534 (1984) oder Chem. Ber. 106, 3275-3290 (1973)):

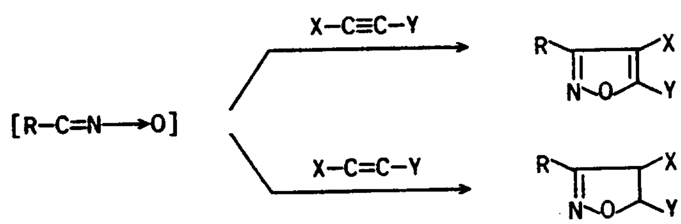


(a)

(b)

X, Y = $COOCH_3$, $COOC_2H_5$

Da die zur Umsetzung benötigten Nitriloxide IV sehr reaktiv und nur einige wenige in Substanz stabile Nitriloxidverbindungen bekannt sind, werden sie in der Regel in situ im Reaktionsgemisch erzeugt, z.B. indem man beispielsweise entsprechende Aldoxime mittels anorganischer Hypochlorite gemäß der folgenden Reaktionsgleichung oxidiert (vgl. DE-A 27 54 832; Synthesis 1982, 508):

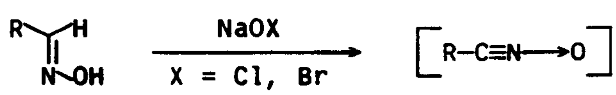


Der Erfindung lag nun die Aufgabe zugrunde, ein vorteilhaftes Verfahren zur Herstellung von Isoxazol-4,5-dicarbonsäure-diestern zu finden, nach dem es vermieden wird, die sehr teuren und aufwendig herzustellenden Acetylendicarbonsäurediester als Ausgangsstoffe zu verwenden. Des weiteren bestand die Aufgabe, neue Isoxazol-4,5-dicarbonsäure-diester zu finden, welche sich u.a. als Zwischenprodukte zur Herstellung von Pflanzenschutzmitteln eignen.

Demgemäß wurde ein Verfahren zur Herstellung von Isoxazol-4,5-dicarbonsäure-diestern der allgemeinen Formel I

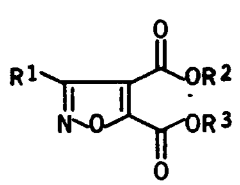

(I),

in der $R^1$ ein beliebiger, unter den Reaktionsbedingungen inerter organischer Rest ist und in der $R^2$ und $R^3$ jeweils für eine Alkyl-, Cycloalkyl- oder Benzylgruppe stehen, gefunden, das dadurch gekennzeichnet ist, daß man ein Aldoxim der allgemeinen Formel II

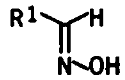

(II)

mit einem Maleinsäurediester oder Fumarsäurediester der allgemeinen Formel III,

$R^2OOC\text{-}C=C\text{-}COOR^3$ (III)

in Gegenwart einer wäßrigen Lösung eines Hypohalogenits umsetzt, wobei das Hypohalogenit im Überschuß, bezogen auf II, vorliegt.

Des weiteren wurden neue Isoxazol-4,5-dicarbonsäure-diester der allgemeinen Formel I'

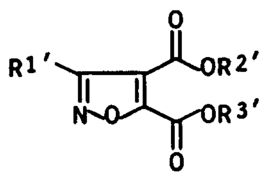

(I'),

in der $R^{1'}$ für eine mit einer $C_1$- bis $C_4$-Acyloxy- oder eine mit einer $C_1$- bis $C_4$-Alkoxycarbonylgruppe substituierte $C_1$- bis $C_4$-Alkylgruppe steht und in der die Reste $R^{2'}$ und $R^{3'}$ $C_1$- bis $C_4$-Alkylgruppen sind, gefunden.

Mit dem erfindungsgemäßen Verfahren ist es erstmals möglich, Isoxazol-4,5-dicarbonsäure-diester I aus den Aldoximen II und Maleinsäurediestern oder Fumarsäurediestern III gemäß folgender Reaktionsgleichung herzustellen:

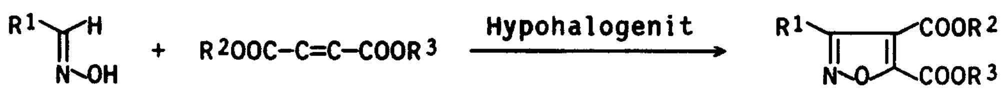


II III I

Als Hypohalogenite werden im erfindungsgemäßen Verfahren im allgemeinen Hyprobromite und Hypochlorite, letztere bevorzugt, verwendet. Es können zu diesem Zweck wäßrige Lösungen der unterchlorigen oder unterbromigen Säure eingesetzt werden, vorzugsweise werden aber Alkalimetall- oder Erdalkalimetall-Hypochlorite oder -Hypobromite, beispielsweise Natriumhypochlorit, Kaliumhypochlorit, Calciumhypochlorit, Magnesiumhypochlorit, Strontiumhypochlorit, Bariumhypochlorit oder die entsprechenden Hypobromite benutzt. Besonders bevorzugt werden Natrium, Kalium- und Calciumhypochlorit, und zwar in Form ihrer handelsüblichen wäßrigen Lösungen, angewandt. Falls solche Hypohalogenitlösungen nicht verfügbar sind, können sie durch Einleiten von Chlor oder Brom in wäßrige Lösungen oder Suspensionen der Hydroxide, Carbonate oder Hydrogencarbonate dieser Metalle hergestellt werden. Es können selbstverständlich auch Mischungen verschiedener Hypohalogenitlösungen im erfindungsgemäßen Verfahren zum Einsatz kommen.

Da die Hypohalogenite im allgemeinen als wäßrige Lösungen zur Reaktionsmischung gegeben werden, die Maleinsäurediester oder Fumarsäurediester sich jedoch in der Regel nicht oder nur zu einem geringen Maße in der wäßrigen Phase lösen, bilden sich bei dieser Zugabe in der Regel zwei Phasen. Deshalb werden diese Ausgangsverbindungen zweckmäßigerweise in einem organischen Lösungsmittel gelöst. Dazu können sowohl Lösungsmittel verwendet werden, die mit der wäßrigen Phase nicht mischbar sind, als auch solche, die sich in beiden Phasen, der organischen und der wäßrigen, lösen und auf diese Art ein homogenes Reaktionsmedium erzeugen. Im allgemeinen ist es zweckmäßig, in einem Zweiphasensystem zu arbeiten.

Für das erfindungsgemäße Verfahren geeignete Lösungsmittel sind z.B. Alkohole, wie Methanol, Ethanol, Propanol oder Isopropanol, Ketone, wie Aceton oder Methylethylketon, Ether wie Diethylether,

Methyl-tert.-butylether, Tetrahydrofuran oder Dioxan, Kohlenwasserstoffe, wie Pentan, Hexan, Cyclohexan, Petrolether, Weißöle oder Ligroin, halogenierte aliphatische Kohlenwasserstoffe, wie Methylenchlorid, Chloroform, Tetrachlorkohlenstoff, Dichlorethan, Trichlorethan, Tetrachlorethan oder Perchlorethan, aromatische Verbindungen wie Benzol, Toluol, Xylole oder Chlorbenzole, Ester, wie Ethylacetat sowie Dimethylformamid, N-Methylpyrrolidon, Dimethylsulfoxid, Sulfolan usw.. Selbstverständlich können auch Lösungsmittelgemische eingesetzt werden.

Werden wasserunlösliche Lösungsmittel benutzt, so kann es sich für den Ablauf und das Ergebnis der Umsetzung vorteilhaft auswirken, wenn dem Reaktionsmedium Phasentransferkatalysatoren wie z.B. quaternäre Ammonium- oder Phosphoniumsalze, beispielsweise Triethylbenzylammoniumchlorid, Trimethylbenzylammoniumbromid, Triphenylbenzylammoniumchlorid, Methyltributylammoniumjodid, Tetrabutylammoniumhydrogensulfat, Tetrabutylammoniumchlorid, Tetrabutylammoniumbromid oder Benzyltributylphosphoniumbromid in Mengen von im allgemeinen 0,1 bis 10 g/l Reaktionsmischung zugesetzt werden. Zweckmäßigerweise wird beim Vorliegen solcher Zwei- oder ggf. Mehrphasensysteme die Reaktionsmischung besonders intensiv gerührt.

Die Temperatur, bei der die Umsetzung durchgeführt wird, kann in weiten Bereichen variiert werden. In der Regel findet die Umsetzung schon bei Temperaturen von -15 °C und tiefer statt, und nach oben wird der Temperaturbereich im Prinzip nur durch den Siedepunkt des verwendeten Lösungsmittels begrenzt, da die Umsetzung Zweckmäßigerweise bei Atmosphärendruck ausgeführt wird. Vorzugsweise wird bei Temperaturen im Bereich von 0 bis 40 °C gearbeitet. Die Reaktion kann auch unter erhöhtem Druck ausgeführt werden, insbesondere unter autogen erzeugtem Druck, bevorzugt ist Atmosphärendruck.

Für das Gelingen des erfindungsgemäßen Verfahrens und zur Vermeidung von Nebenreaktionen ist es oft zweckmäßig, dem Reaktionsmedium Puffersubstanzen zuzufügen, da beispielsweise durch einen hohen Alkaligehalt der Hypochlorit-Lösung (herstellungsbedingt) andernfalls eine Hydrolyse der Estergruppen erfolgen kann. Im allgemeinen reicht es aus, die Reaktion so durchzuführen, daß der pH-wert der wäßrigen Phase im Falle von Zweiphasensystemen im Bereich von etwa pH 5 bis pH 11 liegt, während beim Arbeiten in einer homogenen Reaktionsmischung dieses für den pH-Wert dieser wäßrigorganischen Mischung gilt.

Als Puffersubstanzen können im Prinzip alle Puffersysteme Verwendung finden, welche in der Lage sind, im angegebenen pH-Bereich ihre Pufferwirkung zu entfalten. Vorzugsweise werden jedoch herkömmliche Puffersubstanzen, wie Natriumhydrogencarbonat, Natriumacetat oder das Natriumdihydrogenphosphat/Dinatriumhydrogenphosphat-Puffersystem verwendet. Die Puffersubstanzen können der Reaktionsmischung als Feststoffe zugemischt werden. Zweckmäßigerweise werden aber Pufferlösungen benutzt. Die Stärke der Pufferlösungen kann im Prinzip beliebig gewählt werden, um jedoch nicht mit allzu großen Flüssigkeitsmengen hantieren zu müssen, verwendet man im allgemeinen 0,01 - 1 molare Pufferlösungen.

Zweckmäßigerweise wird der gewünschte pH-Wert der wäßrigen Phase bzw. der wäßrig-organischen Lösung schon vor der Zugabe des Hypohalogenits mit Hilfe von Puffersubstanzen oder Pufferlösungen eingestellt. Anschließend, während der Hypohalogenitzugabe, wird der pH-Wert vorteilhaft kontinuierlich kontrolliert und erforderlichenfalls durch Hinzufügung weiteren Puffers oder von Säuren oder Laugen im gewünschten pH-Bereich gehalten.

Bei der Umsetzung empfindlicher, d.h. besonders reaktionsfähiger Ausgangsverbindungen II und/oder III kann es sich als vorteilhaft erweisen, nur eine dieser Substanzen im gepufferten Medium vorzulegen und den anderen Reaktanten gleichzeitig mit dem Hypohalogenit dazu zudosieren. Eine andere Möglichkeit besteht für diesen Fall darin, einen der Reaktanten II oder III vollständig, den zweiten Reaktanten jedoch nur zu einem geringen Teil, beispielsweise einem Zehntel der benötigten Menge, vorzulegen und den Rest dieses Reaktanten gleichzeitig mit dem Hypohalogenit dem Reaktionsgemisch hinzuzufügen. Vorteilhaft wird die Zugabe der Hypohalogenitlösung so gesteuert, daß in der Reaktionsmischung keine hohe stationäre Konzentration an Hypohalogenit und Nitriloxid entsteht.

Zur Herstellung der Isoxazol-4,5-dicarbonsäure-diester I setzt man zweckmäßigerweise äquimolare Mengen des Aldoxims II und des Maleinsäurediesters oder Fumarsäurediesters III mit dem Hypohalogenit um. In einigen Fällen kann es sich als günstig erweisen, wenn ein überschuß an Maleinsäurediester oder Fumarsäurediester III - beispielsweise 2 Åquivalente bezogen auf die Menge Aldoxim - eingesetzt wird, wobei die nicht verbrauchte Menge der Ausgangskomponente III in der Regel anschließend zurückgewonnen wird.

Das Hypohalogenit wird im Überschuß, bezogen auf das Aldoxim II, verwendet. In der Regel verwendet man mindestens 1,5 mol Hypohalogenit, bezogen auf II, vorteilhaft mindestens 2 Äquivalente Häufig ist es von Vorteil, das Hypohalogenit in einer Menge von 2 bis 3 Äquivalenten, bezogen auf eingesetztes Aldoxim II, zu verwenden.

Aus verfahrenstechnischen Gründen kann es ggf. vorteilhaft sein, den Umsatz durch Verwendung unterstöchiometrischer Mengen an Hypohalogenit - etwa 150 bis 190 mol-% Hypohalogenit pro mol II - zu

begrenzen. Ebenso ist es möglich, mit unter- oder überstöchiometrischen Mengen der Reaktanten II oder III zu arbeiten.

Bei der Durchführung der Umsetzungen geht man in der Regel so vor, daß man alle Komponenten des Reaktionssystems, mit Ausnahme des Hypohalogenits in der wäßrig-organischen Reaktionsmischung vorlegt und dann dieser Mischung unter intensivem Rühren und laufender pH-Überwachung die Hypohalogenitlösung zuführt. Die optimale Geschwindigkeit der Hypohalogenitzugabe richtet sich im allgemeinen nach der Reaktivität der umzusetzenden Reaktanten und wird zweckmäßigerweise in einem Vorversuch ermittelt.

Im übrigen weist das erfindungsgemäße Verfahren keine verfahrenstechnischen Besonderheiten auf, so daß nähere Angaben hierzu entbehrlich sind. Das Verfahren läßt sich mit den üblichen Verfahrenstechniken - Einsatz von Rohrreaktoren oder Rührkesselkaskaden - auch kontinuierlich betreiben. Da sich die Isoxazolderivate I im allgemeinen vorzugsweise in organischen Lösungsmitteln lösen, kann die Aufarbeitung des Reaktionsgemisches und die Isolierung der Isoxazol-4,5-dicarbonsäure-diester in der Regel auf herkömmliche Weise, durch Extraktion, Destillation oder Kristallisation erfolgen. Überschüssiges Hypohalogenit, welches die Aufarbeitung stören oder erschweren kann, läßt sich durch die Zugabe von Reduktionsmitteln wie Eisen(II)sulfat, Thiosulfaten oder Sulfiten zerstören.

Die im erfindungsgemäßen Verfahren benötigten Aldoxime II sind entweder bekannt oder können leicht nach den allgemein bekannten Verfahren (siehe z.B. Houben-Weyl, Methoden der organischen Chemie, Bd. 10/4, Seite 55 bis 66, Thieme, Stuttgart 1968) durch Umsetzung der entsprechenden Aldehyde mit Hydroxylamin hergestellt werden. Die Aldoxime II können selbstverständlich sowohl in Form ihrer E- oder Z-Isomeren als auch als Gemische dieser Stereoisomeren verwendet werden. Die Maleinsäurediester oder Fumarsäurediester III sind Handelsprodukte.

Das erfindungsgemäße Verfahren zur Herstellung der Isoxazol-4,5-dicarbonsäure-diester I ist praktisch universell anwendbar. So können Verbindungen I aus den entsprechenden Aldoximen II erhalten werden, in denen der Rest $R^1$ ein aliphatischer Rest mit 1 bis 20, ein cycloaliphatischer Rest mit 3 bis 10, ein aromatischer Rest mit 6 bis 10, ein heteroaromatischer oder heterocyclischer Rest mit 3 bis 10 oder ein araliphatischer Rest mit 4 bis 12 Kohlenstoffatomen ist. Die Begrenzung der Kohlenstoffzahl der Reste $R^1$ nach oben ist allein durch die Nützlichkeit und Verwendbarkeit der betreffenden Verbindungen begründet und beruht nicht auf einer mangelnden Durchführbarkeit des erfindungsgemäßen Verfahrens bei größeren Resten $R^1$.

Die Reste $R^1$ können weiterhin substituiert sein. Die Art und die Anzahl der Substituienten kann im Prinzip, selbstverständlich unter der Voraussetzung des chemisch möglichen, beliebig gewählt werden, vorausgesetzt, die Substituenten verhalten sich unter den Reaktionsbedingungen gegenüber dem oxidierenden Agens, also der basischen Hypohalogenitlösung, als auch gegenüber dem in situ gebildeten Nitriloxid inert. So können nach dem erfindungsgemäßen Verfahren auch Isoxazolderivate I hergestellt werden, in denen die aliphatischen, araliphatischen oder cycloaliphatischen Reste $R^1$ Acetalgruppen oder Estergruppen enthalten oder in denen die Kohlenstoffketten durch Heteroatome, insbesondere Sauerstoffatome, unterbrochen sind.

Die Art der Reste $R^2$ und $R^3$, welche über den Maleinsäurediester oder Fumarsäurediester in die Verbindung I eingebracht werden, ist im allgemeinen für den Ablauf der Umsetzung nicht kritisch und kann dementsprechend beliebig gewählt werden. Zweckmäßigerweise verwendet man jedoch Alkylgruppen als Reste $R^2$ und $R^3$, insbesondere $C_1$- bis $C_4$-Alkylgruppen. Daneben sind $C_3$-$C_6$-Cycloalkylreste wie Cyclopropyl, Cyclobutyl, Cyclopentyl oder Cyclohexylreste sowie der Benzylrest zu nennen.

Die Reste $R^2$ und $R^3$ können gleich oder verschieden sein. Sind die Reste $R^2$ und $R^3$ verschieden, entsteht bei der Cycloaddition des Nitriloxids mit dem Maleinsäurediester oder Fumarsäurediester III als Produkt im allgemeinen ein Gemisch der Regioisomeren Ia und Ib,

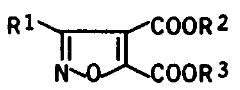

Ia

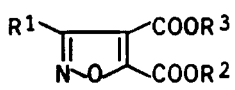

Ib

dessen Zusammensetzung bezüglich der einzelnen Regioisomeren im wesentlichen durch die sterischen Anforderungen der jeweiligen Reste $R^1$, $R^2$ und $R^3$ bestimmt wird. Dieser Effekt ist, je nach dem, wie die als Zwischenprodukte dienenden Verbindungen weiterverarbeitet werden sollen, nicht kritisch und kann unter Umständen sogar erwünscht sein. Im allgemeinen werden jedoch Maleinsäurediester oder Fumarsäurediester III, in denen die Reste $R^2$ und $R^3$ gleich sind, bevorzugt zu den Verbindungen I umgesetzt.

Vorteilhaft können nach dem erfindungsgemäßen Verfahren Isoxazol-4,5-dicarbonsäurediester I hergestellt werden, in denen der Rest $R^1$ eine $C_1$-bis $C_{10}$, insbesondere eine $C_1$- bis $C_6$-Alkylgruppe, eine $C_3$ bis $C_8$-, insbesondere eine $C_3$- bis $C_7$-Cycloalkylgruppe, ein 5- bis 7-gliedriger, ein oder zwei der Heteroatome Sauerstoff oder Stickstoff enthaltender, aromatischer oder cycloaliphatischer Heterocyclus oder eine Phenyl- oder Benzylgruppe ist.

Diese Reste $R^1$ können unsubstituiert sein oder unter den Reaktionsbedingungen interte Substituenten tragen.

So können die Alkylgruppen $R^1$, je nach ihrer Größe 1, 2, 3, 4 oder 5, vorzugsweise bis zu 3, gleiche oder verschiedene Substituenten, wie $C_3$- bis $C_7$-Cycloalkyl-, $C_1$- bis $C_3$-Alkoxy-, Alkoxycarbonyl-, Acyloxy-, -Dialkoxy-, Halogen, Cyano- oder Phenylgruppen tragen, wobei die Phenylsubstituenten wiederum mit bis zu 3, vorzugsweise mit einem oder zweien der Substituenten Halogen, $C_1$- bis $C_4$-Alkyl, $C_1$- bis $C_4$-Halogenalkyl, $C_1$- bis $C_4$-Alkoxy, -Acyloxy, -Alkoxycarbonyl, $C_1$- bis $C_4$-Halogenalkoxy, Cyano oder Nitro substituiert sein können und wobei das Substitutionsmuster dieser Phenylsubstituenten im allgemeinen nicht kritisch ist.

Die Alkylgruppen $R^1$ können geradkettig oder verzweigt sein. Besonders vorteilhaft können erfindungsgemäß die Verbindungen 1, in denen $R^1$ eine Alkylgruppe ist, welche mit den Substituenten $C_1$- bis $C_4$-Alkoxy, -Acyloxy, Alkoxycarbonyl, Halogen und/oder Phenyl substituiert ist, wobei der Phenylsubstituent als Substituenten vorzugsweise Halogenatome und/oder $C_1$- bis $C_4$-Alkylgruppen trägt, hergestellt werden.

Es ist für den Fachmann selbstverständlich, daß die Anzahl der Substituenten von der Anzahl der Kohlenstoffatome im aliphatischen Rest abhängig ist. Das Substitutionsmuster der aliphatischen Reste $R^1$ ist im allgemeinen für die erfindungsgemäße Umsetzung nicht kritisch.

Die Cycloalkylgruppen $R^1$ können je nach ihrer Größe mit 1, 2, 3, 4 oder 5, vorzugsweise mit bis zu 3, gleichen oder verschiedenen $C_1$- bis $C_4$-Alkyl-, $C_1$- bis $C_3$-Alkoxy-, -Acyloxy-, -Alkoxycarbonyl- oder Halogensubstituenten substituiert sein.

Nach dem erfindungsgemäßen verfahren können vorteilhaft auch Isoxazol-4,5-dicarbonsäure-diester produziert werden, in denen der Rest $R^1$ ein 5- bis 7-gliedriger Heterocyclus ist, der mit 1 bis 3 gleichen oder verschiedenen $C_1$- bis $C_4$-Alkyl-, $C_1$- bis $C_4$-Alkoxy-, $C_1$- bis $C_4$-Alkoxycarbonyl-, -Acyloxy und/oder, besonders bevorzugt, mit $C_1$- bis $C_4$-Alkylgruppen substituiert sein kann. Der Heterocyclus $R^1$ kann aromatischer oder cycloaliphatischer Natur sein. Die heteroaromatischen Reste $R^1$ können 1 oder 2 der Heteroatome Sauerstoff und/oder Stickstoff enthalten. Nach dem erfindungsgemäßen Verfahren können beispielsweise die Isoxazolderivate I, in denen der Rest $R^1$ für eine substituierte oder unsubstituierte Pyridyl-, Pyrazinyl-, Pyrimidinyl-, Pyridazinyl-, Pyrazolyl-, Imidazolyl-, Oxazolyl- oder Isoxazolylgruppe steht, vorteilhaft hergestellt werden.

Ebenfalls vorteilhaft lassen sich erfindungsgemäße Isoxazolderivate I darstellen, in denen $R^1$ ein 5- bis 6-gliedriger Cycloaliphat ist, der 1 oder 2 der Heteroatome Stickstoff und/oder bevorzugt Sauerstoff enthält. Als Beispiele für solche heterocycloaliphatischen Reste $R^1$ seien substituierte oder unsubstituierte Tetrahydrofuranyl-, Tetrahydropyranyl-, 1,3-Dioxolanyl-, 1,3-Dioxanyl-, 1,4-Dioxanyl-, Oxepanyl-, 1,3-Dioxepanyl-, 1,4-Dioxepanyl-, 1,5-Dioxepanyl-, Pyrrolidinyl-, Imidazolidinyl-, Piperidinyl- oder Piperazinylgruppen genannt.

Weiterhin lassen sich nach dem erfindungsgemäßen Verfahren vorteilhaft Isoxazolderivate I herstellen, in denen $R^1$ für eine substituierte oder unsubstituierte $C_6$- bis $C_{10}$-Aryl- oder eine $C_7$- bis $C_{12}$-Aralkylgruppe, insbesondere die Phenyl- oder die Benzylgruppe steht. Die Arylreste, insbesondere die Phenylgruppe, können dabei 1, 2 oder 3 gleiche oder verschiedene $C_1$- bis $C_6$-Alkyl-, $C_1$- bis $C_6$-Halogenalkyl-, $C_1$- bis $C_6$-Alkoxy-, -Alkoxycarbonyl-, -Acyloxy-, $C_1$- bis $C_6$-Halogenalkoxy-, Halogen-, Nitro- oder Cyanogruppen als Substituenten tragen.

Nach dem erfindungsgemäßen Verfahren können insbesondere auch die neuen Isoxazol-4,5-dicarbonsäure-diester der allgemeinen Formel I'

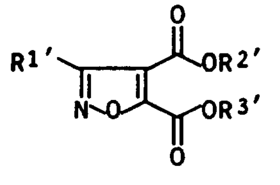

(I')

vorteilhaft hergestellt werden, in denen $R^{1'}$ für eine mit einer $C_1$- bis $C_4$-Acyloxy- oder eine mit einer $C_1$- bis $C_4$-Alkoxycarbonylgruppe substituierte $C_1$- bis $C_4$-Alkylgruppe steht und in der die Reste $R^{2'}$ und $R^{3'}$ $C_1$- bis $C_4$-Alkylgruppen sind. Die Umsetzung von Isoxazol-4,5-dicarbonsäureestern zu herbizid wirksamen Isoxazol-5-carbonsäureamiden ist z.B. in DE-A 38 12 225 beschrieben.

Beispiel 1

Eine Mischung aus 18,3 g (0,21 mol) Isobutyraldoxim, 7,1 g (0,04 mol) Dinatriumhydrogenphosphat-Dihydrat, 100 ml Methylenchlorid, 28,8 g (0,2 mol) Dimethylfumarat und 100 ml Wasser wird auf 10 °C gekühlt. Unter intensivem Rühren werden zu dieser Mischung 267 g einer 13,4 %igen Natriumhypochlorit-Lösung (0,48 mol) innerhalb 2 Stunden zugetropft, anschließend wird bei 20 °C 15 Stunden nachgerührt. Nach Abtrennen der organischen Phase wird die wäßrige Phase mit Methylenchlorid extrahiert, anschließend werden die organischen Phasen vereinigt, über Natriumsulfat getrocknet und im Vakuum eingeengt. Das Rohprodukt wird durch eine Destillation gereinigt.

Man erhält 30 g 3-Isopropyl-isoxazol-4,5-dicarbonsäure-dimethylester, Sdp.: 102 °C/0,4 mbar. Ausbeute: 66 % der Theorie

$^1$H-NMR ($CDCl_3$): 1,4(d,6H);3,2-3,5(m,1H);3,9(s,3H);4,0(s,3H).

Beispiel 2

Eine Mischung aus 18,3 g (0,21 mol) Isobutyraldoxim, 1,11 g (0,004 mol) Tetrabutylammoniumchlorid, 3,5 g (0,02 mol) Dinatriumhydrogenphosphat-Dihydrat, 3,1 g (0,02 mol) Natriumdihydrogenphosphat-Dihydrat, 28,8 g (0,2 mol) Dimethylfumarat, 100 ml Methylenchlorid und 100 ml Wasser wird auf 10 °C gekühlt. Innerhalb zwei Stunden werden unter intensivem Rühren 278 g einer 13,4 %igen Natriumhypochlorit-Lösung (0,5 mol) zugetropft, anschließend wird noch zwei Stunden bei 20 °C gerührt.

Die Aufarbeitung und Reinigung erfolgt wie in Beispiel 1.

Ausbeute an 3-Isopropyl-isoxazol-4,5-dicarbonsäure-dimethylester: 38 g, entsprechend 84 % der Theorie.

Beispiel 3

Eine Mischung aus 87 g (1,0 mol) Isobutyraldoxim, 17,8 g (0,1 mol) Dinatriumhydrogenphosphat-Dihydrat, 15,6 g Natriumdihydrogenphosphat-Dihydrat, 8,3 g (0,03 mol) Tetrabutylammoniumchlorid, 288 g (2,0 mol) Dimethylmaleinat, 500 ml Methylenchlorid und 500 ml Wasser wird auf 10 °C gekühlt. Innerhalb 4 Stunden werden unter intensivem Rühren 1 390 g einer 13,4 %igen Natriumhypochlorit-Lösung zugetropft, anschließend wird noch 16 Stunden bei 20 °C gerührt.

Die organische Phase wird abgetrennt, die wäßrige Phase wird zweimal mit Methylenchlorid extrahiert, die vereinigten organischen Phasen werden über Natriumsulfat getrocknet und im Vakuum eingeengt.

Nach Destillation erhält man 184 g 3-Isopropyl-isoxazol-4,5-dicarbonsäure-dimethylester, Sdp.: 99 °C/0,3 mbar (81 % der Theorie). In einer Vorfraktion sind 140 g Maleinsäuredimethylester (0,97 mol), der in die Reaktion rückgeführt werden kann, enthalten.

Beispiele 4 bis 21

Allgemeine Vorschrift:

Eine Mischung aus 0,5 mol Aldoxim, 6,9 g (0,025 mol) Tetrabutylammoniumchlorid, 8,9 g (0,05 mol) Dinatriumhydrogenphosphat-Dihydrat, 7,8 g (0,05 mol) Natriumdihydrogenphosphat-Dihydrat, 75,6 g (0,525 mol) Dimethylfumarat, 250 ml Methylenchlorid und 250 ml Wasser wird bei 10 °C vorgelegt. Unter intensivem Rühren werden 667 g einer 13,4 %igen Natriumhypochlorit-Lösung (1,2 mol) innerhalb 3 Stunden zugetropft, anschließend wird noch 16 Stunden bei 20 °C gerührt.

Die organische Phase wird abgetrennt, die wässrige Phase wird zweimal mit Methylenchlorid extrahiert, die vereinigten organischen Phasen werden dreimal mit Wasser gewaschen, über Natriumsulfat getrocknet und im Vakuum eingeengt.

Die Rohprodukte werden im Falle von leicht flüchtigen Verbindungen durch Destillation gereinigt. Bei nicht destillierbaren Produkten werden die Niedersieder durch Andestillation abgetrennt, wobei die Verfahrensprodukte in der Regel in einer Reinheit über 95 % (gaschromatographisch bestimmt) erhalten werden.

Die Ergebnisse der Umsetzungen sind in der Tabelle aufgelistet. Diese Tabelle enthält außer der Aufzählung der hergestellten Isoxazolderivate I Angaben zur Ausbeute, zum Schmelzpunkt (Fp), falls diese Verbindungen kristallin enthalten wurden bzw. zum Siedepunkt (Kp), falls diese Produkte destilliert wurden, sowie eine Wiedergabe der wichtigsten Daten der 250 MHZ $^1$H-NMR-Spektren dieser Verbindungen in

Deuterochloroform ($CDCl_3$).

In der Tabelle werden folgende Abkürzungen benutzt:

Me : Methyl-;
Et : Ethyl;
iPr : Isopropyl;
c-Pr: Cyclopropyl;
t-Bu: tert.-Butyl;
Ph : Phenyl;
s : Singulett;
d : Dublett;
t : Triplett;
q : Quadruplett;
m : Multiplett

Tabelle: Isoxazol-4,5-dicarbonsäureester

| Beispiel | $R^1$ | $R^2=R^3$ | Ausbeute | Fp/Kp | $^1$H-NMR ($CDCl_3$) |
|---|---|---|---|---|---|
| 4 | Me | Me | 78 % | 34-35°C | 2,5(s,3H),3,9(s,3H),4,0(s,3H). |
| 5 | Et | Me | 83 % | 115°C/0,5 mbar | 1,3(t,3H),2,9(q,2H),3,9(s,3H),4,0(s,3H). |
| 6 | c-Pr | Me | 75 % | | 0,9-1,2(m,4H),2,1-2,4(m,1H),3,9(s,3H),4,0(s,3H). |
| 7 | t-Bu | Me | 89 % | 99-101°C/0,3 mbar | 1,4(s,9H),3,9(s,3H),3,95(s,3H). |
| 8 | 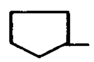 | Me | 82 % | | 1,6-2,2(m,8H),3,3-3,5(m,1H),3,9(s,3H),4,0(s,3H). |
| 9 | 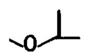 | Me | 58 % | 120°C/0,2 mbar | 1,6(d,3H),3,35(s,3H),3,9(s,3H),4,0(s,3H), 4,7(q,1H). |
| 10 | 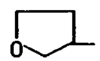 | Me | 63 % | | 1,8-2,5(m,2H),3,9(s,3H),4,0(s,3H),3,7-4,3(m,5H). |
| 11 | 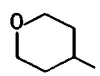 | Me | 80 % | | 1,8-2,1(m,4H),3,2-3,4(m,1H),3,4-3,6(m,2H), 3,9(s,3H),4,0(s,3H),4,0-4,2(m,2H). |
| 12 | 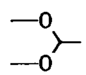 | Me | 45 % | | 3,5(s,6H),3,9(s,3H),4,0(s,3H),5,7(s,1H). |
| 13 | 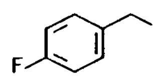 | Me | 78 % | | 3,8(s,3H),4,0(s,3H),4,2(s,2H),6,9-7,1(m,2H), 7,1-7,4(m,2H). |
| 14 | Ph | Me | 90 % | | 3,8(s,3H),4,0(s,3H), 7,3-7,6(m,3H),7,6-7,8(m,2H). |
| 15 | 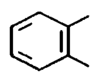 | Me | 85 % | | 2,3(s,3H),3,8(s,3H),4,0(s,3H),7,2-7,5(m,4H). |

Tabelle (Fortsetzung)

| Beispiel | $R^1$ | $R^2$=$R^3$ | Ausbeute | Fp/Kp | $^1H$-NMR ($CDCl_3$) |
|---|---|---|---|---|---|
| 16 | 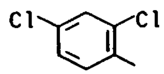 | Me | 85 % | | 3,8(s,3H),4,0(s,3H),7,3-7,6(m.3H). |
| 17 | 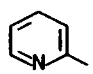 | Me | 59 % | | 3,95(s,3H),4,0(s,3H),7,4(m,1H),7,8(dt,1H), 8,1(d,1H),8,7(d,1H). |
| 18 | 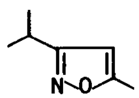 | Me | 61 % | | 1,3(d,6H),3,0-3,2(m,1H),3,9(s,3H),4,0(s,3H), 6,9(s,1H). |
| 19 | 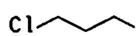 | Me | 85 % | | 2,1-2,3(m,2H),3,1(t,2H),3,6(t,2H),3,9(s,3H), 4,0(s,3H). |
| 20 | 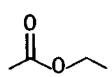 | Me | 48 % | | 2,1(s,3H),3,9(s,3H),4,0(s,3H),5,4(s,2H). |
| 21 | 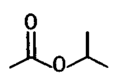 | Me | 59 % | | 1,7(d,3H),2,1(s,3H),3,9(s,3H),4,0(s,3H),6,2(q,1H). |

## Patentansprüche

1. Verfahren zur Herstellung von Isoxazol-4,5-dicarbonsäure-diestern der allgemeinen Formel I

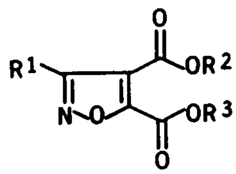

(I),

in der $R^1$ ein beliebiger, unter den Reaktionsbedingungen inerter organischer Rest ist und in der $R^2$ und $R^3$ jeweils für eine Alkyl-, Cycloalkyl- oder Benzylgruppe stehen, dadurch gekennzeichnet, daß man ein Aldoxim der allgemeinen Formel II

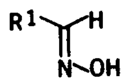

(II)

mit einem Maleinsäurediester oder Fumarsäurediester der allgemeinen Formel III

$R^2OOC\text{-}C = C\text{-}COOR^3$ (III),

in der $R^2$ und $R^3$ gleich oder verschieden sind und die o.g. Bedeutung haben, in Gegenwart einer wäßrigen Lösung eines Hypohalogenits umsetzt, wobei das Hypohalogenit im Überschuß, bezogen auf II, vorliegt.

2. Verfahren nach Anspruch 1, dadurch gekennzeichnet, daß $R^1$ ein aliphatischer Rest mit 1 bis 20, ein cycloaliphatischer Rest mit 3 bis 10, ein aromatischer Rest mit 6 bis 10, ein heteroaromatischer oder heterocyclischer Rest mit 3 bis 10 oder ein araliphatischer Rest mit 4 bis 12 Kohlenstoffatomen ist, wobei diese Reste mit unter den Reaktionsbedingungen inerten Substituenten substituiert sein können.

3. Verfahren nach den Ansprüchen 1 und 2, dadurch gekennzeichnet, daß man als Hypohalogenit Natriumhypochlorit, Kaliumhypochlorit und/oder Calciumhypochlorit verwendet.

4. Verfahren nach den Ansprüchen 1 bis 3, dadurch gekennzeichnet, daß man 2 bis 3 mol des Hypohalogenits pro Mol Aldoxim II verwendet.

5. Verfahren nach den Ansprüchen 1 bis 4, dadurch gekennzeichnet, daß man die Umsetzung in einem organisch-wäßrigen Zweiphasensystem durchführt.

6. Verfahren nach den Ansprüchen 1 bis 5, dadurch gekennzeichnet, daß man die Umsetzung bei Temperaturen von 0 bis 40 °C durchführt.

7. Verfahren nach den Ansprüchen 1 bis 5, dadurch gekennzeichnet, daß man die Umsetzung im pH-Bereich von pH 5 bis 11 vornimmt.

8. Isoxazol-4,5-dicarbonsäure-diester der allgemeinen Formel I'

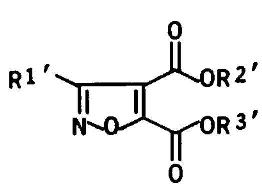

(I'),

in der R1' für eine mit einer $C_1$- bis $C_4$-Acyloxy- oder eine mit einer $C_1$-bis $C_4$-Alkoxycarbonylgruppe substituierte $C_1$- bis $C_4$-Alkylgruppe steht und in der die Reste $R^{2'}$ und $R^{3'}$ $C_1$- bis $C_4$-Alkylgruppen sind.

## Claims

**1.** A process for preparing an isoxazole-4,5-dicarboxylic diester of the formula I

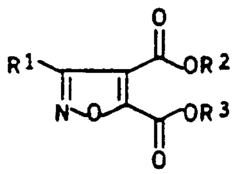

(I)

where $R^1$ is any organic radical which is inert under the reaction conditions, and $R^2$ and $R^3$ are each alkyl, cycloalkyl or benzyl, which comprises reacting an aldoxime of the formula II

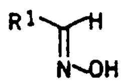

(II)

with a diester of maleic or fumaric acid, of the formula III

$R^2OOC\text{-}C = C\text{-}COOR^3$ (III)

where $R^2$ and $R^3$ are identical or different and have the abovementioned meanings, in the presence of an aqueous solution of a hypohalite which is present in excess relative to II.

**2.** A process as claimed in claim 1, wherein $R^1$ is an aliphatic radical with 1 to 20, a cycloaliphatic radical with 3 to 10, an aromatic radical with 6 to 10, a heteroaromatic or heterocyclic radical with 3 to 10 or an araliphatic radical with 4 to 12 carbon atoms, it being possible for these radicals to be substituted by substituents which are inert under the reaction conditions.

**3.** A process as claimed in claims 1 and 2, wherein sodium hypochlorite, potassium hypochlorite and/or calcium hypochlorite is used as hypohalite.

**4.** A process as claimed in claims 1 to 3, wherein 2 to 3 mol of the hypohalite are used per mole of aldoxime II.

**5.** A process as claimed in claims 1 to 4, wherein the reaction is carried out in an organic/aqueous two-phase system.

**6.** A process us claimed in claims 1 to 5, wherein the reaction is carried out at from 0 to 40 °C.

**7.** A process as claimed in claims 1 to 5, wherein the reaction is carried out at a pH in the range from 5 to 11.

**8.** An isoxazole-4,5-dicarboxylic diester of the formula I'

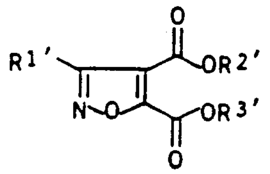

(I')

where $R^{1'}$ is $C_1$-$C_4$-alkyl which is substituted by $C_1$-$C_4$-acyloxy or $C_1$-$C_4$-alkoxycarbonyl, and $R^{2'}$ and $R^{3'}$ are each $C_1$-$C_4$-alkyl.

## Revendications

**1.** Procédé de préparation de diesters d'acide isoxazol-4,5-dicarboxylique de formule générale I

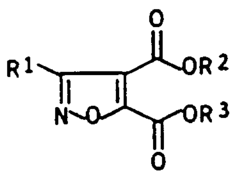

(I),

dans laquelle $R^1$ est un reste organique arbitraire inerte dans les conditions de réaction et dans laquelle $R^2$ et $R^3$ sont mis chacun pour un groupe alkyle, cycloalkyle ou benzyle, caractérisé par le fait que l'on fait réagir une aldoxime de la formule générale II

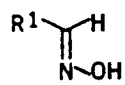

(II)

avec un diester d'acide maléique ou diester d'acide fumarique de la formule générale III

$R^2OOC\text{-}C=C\text{-}COOR^3$ (III),

dans laquelle $R^2$ et $R^3$ sont identiques ou différents et ont la signification sus-indiquée, en présence d'une solution aqueuse d'un hypohalogénite, l'hypohalogénite se trouvant en excès, par rapport à II.

**2.** Procédé selon la revendication 1, caractérisé par le fait que $R^1$ est un reste aliphatique de 1 à 20, un reste cycloaliphatique de 3 à 10, un reste aromatique de 6 à 10, un reste hétéroaromatique, hétérocyclique de 3 à 10 ou un reste araliphatique de 4 à 12 atomes de carbone, ces restes pouvant être substitués avec des substituants inertes dans les conditions de réaction.

**3.** Procédé selon les revendication 1 et 2, caractérisé par le fait que l'on utilise, comme hypohalogénite, l'hypochlorite de sodium, l'hypochlorite de potassium et/ou l'hypochlorite de calcium.

**4.** Procédé selon les revendications 1 à 3, caractérisé par le fait que l'on utilise 2 à 3 moles d'hypohalogénite par mole d'aldoxime II.

**5.** Procédé selon les revendication 1 à 4, caractérisé par le fait que l'on effectue la réaction dans un système à deux phases organique-aqueuse.

**6.** Procédé selon les revendications 1 à 5, caractérisé par le fait que l'on effectue la réaction à des températures de 0 à 40 °C.

7. Procédé selon les revendications 1 à 5, caractérisé par le fait que l'on opère la réaction dans un domaine de pH de 5 à 11.

8. Diester d'acide isoxazol-4,5-dicarboxylique de la formule générale I'

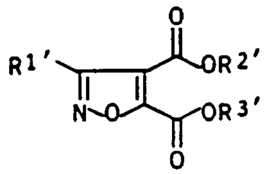

(I'),

dans laquelle $R^{1'}$ est mis pour un groupe acyloxy en C1-C4 ou un groupe alkyle en C1-C4 substitué avec un groupe (alcoxy en C1-C4)-carbonyle et dans laquelle les restes $R^{2'}$ et $R^{3'}$ sont des groupes alkyle en C1-C4.